(19) Europäisches Patentamt
European Patent Office
Office européen des brevets

(11) Veröffentlichungsnummer: **0 121 716**
**B1**

(12) # EUROPÄISCHE PATENTSCHRIFT

(45) Veröffentlichungstag der Patentschrift: **09.12.87**

(21) Anmeldenummer: **84102011.8**

(22) Anmeldetag: **27.02.84**

(51) Int. Cl.4: **C 07 D 409/14**, C 07 D 491/056, A 61 K 31/44 // (C07D409/14, 209:14, 211:70, 333:20)

(54) **Indolderivate.**

(30) Priorität: **11.03.83 DE 3308668**

(43) Veröffentlichungstag der Anmeldung: **17.10.84 Patentblatt 84/42**

(45) Bekanntmachung des Hinweises auf die Patenterteilung: **09.12.87 Patentblatt 87/50**

(84) Benannte Vertragsstaaten: **AT BE CH DE FR GB IT LI NL SE**

(56) Entgegenhaltungen:
**DE - A - 2 827 874**
**FR - A - 2 470 128**
**US - E - 28 973**

**CHEMICAL ABSTRACTS, vol. 94, nr. 9, 2. März 1981, Columbus, Ohio, USA, C. MINOT, P. ROLAND-GOSSELIN, C. THAL "Photocyclization of stilbenes and of related indolic compounds: contribution of the use of reactivity indexes", Seite 657, Zusammenfassung Nr. 64 902 u**
**CHEMICAL ABSTRACTS, vol. 94, no. 7, 16. Februar 1981, Columbus, Ohio, USA, C. GUEREMY, F. AUDIAU, A. CHAMPSEIX, A. UZAN, G. LE FUR, J. RATAUD "3-(4-Piperidinylalkyl)indoles, selective inhibitors of neuronal 5-hydroxytryptamine uptake", Seite 13, Zusammenfassung Nr. 41 139 m**

(73) Patentinhaber: **Merck Patent Gesellschaft mit beschränkter Haftung, Frankfurter Strasse 250, D-6100 Darmstadt (DE)**

(72) Erfinder: **Gottschlich, Rudolf, Dr., Buchenweg 1, D-6107 Reinheim (DE)**
Erfinder: **Böttcher, Henning, Dr., Soderstrasse 95, D-6100 Darmstadt (DE)**
Erfinder: **Hausberg, Hans-Heinrich, Dr., Odenwaldstrasse 30, D-6105 Ober-Ramstadt (DE)**
Erfinder: **Minck, Klaus-Otto, Dr., Büchestrasse 8, D-6105 Ober-Ramstadt (DE)**
Erfinder: **Seyfried, Christoph, Dr., Mathildenstrasse 6, D-6104 Seeheim-Jugenheim (DE)**

(56) Entgegenhaltungen: (Fortsetzung)
**CHEMICAL ABSTRACTS, vol. 94, no. 17, 27. April 1981, Columbus, Ohio, USA, KURARAY Co., LTD., INSTITUTE OF PHYSICAL AND CHEMICAL RESEARCH "Indole derivatives as fungicides", Seite 261, Zusammenfassung Nr. 134 148 d**

**EP 0 121 716 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft neue Indolderivate der allgemeinen Formel I

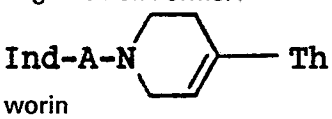

I

worin

Ind einen Indol-3-ylrest, der ein- oder zweifach durch Alkyl, O-Alkyl, S-Alkyl, SO-Alkyl, $SO_2$-Alkyl, OH, F, Cl, Br, $CF_3$ und/oder CN oder durch eine Methylendioxygruppe substituiert sein kann,

A $-(CH_2)_4-$ oder $-CH_2-L-CH_2CH_2-$,

L $-S-$, $-SO-$ oder $-SO_2-$ und

Th einen 2- oder 3-Thienylrest

bedeuten und

worin die Alkylgruppen jeweils 1–4 C-Atome besitzen, sowie deren physiologisch unbedenkliche Säureadditionssalze.

Ähnliche Verbindungen, die Anti-Parkinson-Wirkungen zeigen, sind aus DE-A-2827874 und aus US-E-28973 bekannt. Diese bekannten Verbindungen enthalten jedoch keine Thienylreste.

Der Erfindung lag die Aufgabe zugrunde, neue Verbindungen aufzufinden, die zur Herstellung von Arzneimitteln verwendet werden können. Diese Aufgabe wurde durch die Bereitstellung der Verbindungen der Formel 1 gelöst.

Es wurde gefunden, dass die Verbindungen der Formel 1 und ihre physiologisch unbedenklichen Säureadditionssalze wertvolle pharmakologische Eigenschaften besitzen. So zeigen sie insbesondere Wirkungen auf das Zentralnervensystem, vor allem Dopamin-stimulierende (Anti-Parkinson) Wirkungen. Im einzelnen induzieren die Verbindungen der Formel 1 contralaterales Drehverhalten in Hemiparkinson-Ratten (feststellbar nach der Methode von Ungerstedt et al., Brain Res. 24, (1970), 485–493) und hemmen die Bindung von tritierten Dopaminagonisten und -antagonisten an striäre Rezeptoren (feststellbar nach der Methode von Schwarcz et al., J. Neurochemistry, 34 (1980), 772–778 und Creese et al., European J. Pharmacol., 46 (1977), 377–381). Zusätzlich hemmen die Verbindungen den Zungen-Kieferreflex bei der narkotisierten Ratte (feststellbar in Anlehnung an die Methode von Barnett et al., European J. Pharmacol. 21, (1973), 178–182, und von Ilhan et al., European J. Pharmacol. 33, (1975), 61–64. Weiterhin treten analgetische Wirkungen auf.

Verbindungen der Formel I und ihre physiologisch unbedenklichen Säureadditionssalze können daher als Arzneimittelwirkstoffe und auch als Zwischenprodukte zur Herstellung anderer Arzneimittelwirkstoffe verwendet werden.

Gegenstand der Erfindung sind die Indolderivate der Formel I sowie ihre physiologisch unbedenklichen Säureadditionssalze.

In den Resten Ind bedeutet Alkyl vorzugsweise Methyl, ferner auch Ethyl, n-Propyl, Isopropyl, n-Butyl, Isobutyl, sek.-Butyl oder tert.-Butyl. O-Alkyl ist vorzugsweise Methoxy, ferner auch Ethoxy, n-Propoxy, Isopropoxy, n-Butoxy, Isobutoxy, sek.-Butoxy oder tert.-Butoxy. S-Alkyl steht vorzugsweise für Methylthio, aber auch für Ethylthio, n-Propylthio, Isopropylthio, n-Butylthio, Isobutylthio, sek.-Butylthio oder tert.-Butylthio. SO-Alkyl steht vorzugsweise für Methylsulfinyl, ferner auch für Ethylsulfinyl, n-Propylsulfinyl, Isopropylsulfinyl, n-Butylsulfinyl, Isobutylsulfinyl, sek.-Butylsulfinyl oder tert.-Butylsulfinyl. $SO_2$-Alkyl ist vorzugsweise Methylsulfonyl, ferner auch Ethylsulfonyl, n-Propylsulfonyl, Isopropylsulfonyl, n-Butylsulfonyl, Isobutylsulfonyl, sek.-Butylsulfonyl oder tert.-Butylsulfonyl.

Der Rest Ind bedeutet insbesondere einen unsubstituierten Indol-3-ylrest. Falls Ind jedoch einen substituierten Indol-3-ylrest bedeutet, so ist er vorzugsweise einfach, insbesondere in der 2-, 5- oder 6-Stellung substituiert. Weiterhin ist eine Substitution in 1-, 4- oder 7-Stellung möglich. Bevorzugte disubstituierte Indol-3-ylreste sind in 5, 6-Stellung substituiert; Disubstitution ist auch in 1,2-, 1,4-, 1,5-, 1,6-, 1,7-, 2,4-, 2,5-, 2,6-, 2,7-, 4,5-, 4,6-, 4,7-, 5,7- oder 6,7-Stellung möglich. In allen diesen Fällen können die Substituenten gleich oder verschieden sein.

Im einzelnen sind die bevorzugten Substituenten im Benzolring des Restes Ind Methyl, Ethyl, Methoxy, Ethoxy, Methylthio, Ethylthio, OH, F, Cl, Br, $CF_3$ und CN. Einige bevorzugte Bedeutungen des Restes Ind sind dementsprechend Indol-3-yl, ferner 1-, 2-, 4-, 5-, 6- oder 7-Methylindol-3-yl, 1-, 2-, 4-, 5-, 6- oder 7-Ethyl-indol-3-yl, 4-, 5-, 6- oder 7-Methoxyindol-3-yl, 4-, 5-, 6- oder 7-Ethoxyindol-3-yl, 4-, 5,- 6- oder 7-Methylthioindol-3-yl, 4-, 5-, 6- oder 7-Ethylthioindol-3-yl, 4-, 5-, 6- oder 7-Methylsulfinylindol-3-yl, 4-, 5-, 6- oder 7-Methylsulfonylindol-3-yl, 4-, 5-, 6- oder 7-Hydroxyindol-3-yl, 4-, 5-, 6- oder 7-Fluorindol-3-yl, 4-, 5-, 6- oder 7-Chlorindol-3-yl, 4-, 5-, 6- oder 7-Bromindol-3-yl, 4-, 5-, 6- oder 7-Trifluormethyl-indol-3-yl, 4-, 5-, 6- oder 7-Cyanindol-3-yl, 1,2-, 1,4-, 1,5-, 1,6-, 1,7-, 2,4-, 2,5-, 2,6-, 2,7-, 4,5-, 4,6-, 4,7-, 5,6-, 5,7- oder 6,7-Dimethylindol-3-yl, 1-Methyl-4-, -5-, -6- oder -7-methoxyindol-3-yl, 1-Methyl-4-, -5-, -6- oder -7-fluorindol-3-yl, 1-Methyl-4-, -5-, -6- oder -7-chlor-indol-3-yl, 1-Methyl-4-, -5-, -6- oder -7-bromindol-3-yl, 1-Methyl-4-, -5-, -6- oder -7-trifluormethyl-indol-3-yl, 1-Methyl-4-, -5-, -6- oder -7-cyanindol-3-yl, 2-Methyl-4-, -5-, -6- oder -7-methoxyindol-3-yl, 2-Methyl-4-, -5-, -6- oder -7-methylthioindol-3-yl, 2-Methyl-4-, -5-, -6- oder -7-fluorindol-3-yl, 2-Methyl-4-, -5-, -6- oder -7-chlorindol-3-yl, 2-Methyl-4-, -5-, -6- oder -7-bromindol-3-yl, 2-Methyl-4-, -5-, -6- oder -7-trifluormethylindol-3-yl, 2-Methyl-4-, -5-, -6- oder -7-cyanindol-3-yl, 4-Methyl-5-fluorindol-3-yl, 5-Fluor-6- oder -7-methylindol-3-yl, 4-Methyl-5-chlorindol-3-yl, 4-Chlor-5-methylindol-3-yl, 5-Methyl-6- oder 7-chlorindol-3-yl, 5-Chlor-6- oder -7-methylindol-3-yl, 4,5-, 4,6-, 4,7-, 5,6-, 5,7- oder 6,7-Dimethoxyindol-3-yl, 4,5-, 4,6-, 4,7-, 5,6-, 5,7- oder 6,7-Dichlorindol-3-yl, 4-Trifluormethyl-5-, -6- oder -7-chlorindol-3-yl.

Die Gruppe A ist vorzugsweise $-(CH_2)_4-$. Die

Gruppe L steht vorzugsweise für ein S-Atom. Th ist vorzugsweise ein 2-Thienylrest.

Dementsprechend sind Gegenstand der Erfindung insbesonder diejenigen Verbindungen der Formel I, in denen mindestens einer der genannten Reste eine der vorstehend angegebenen, insbesondere der vorstehend angegebenen bevorzugten Bedeutungen hat. Einige bevorzugte Gruppen von Verbindungen können durch die folgenden Teilformeln Ia bis Ik ausgedrückt werden, die der Formel I entsprechen und worin die nicht näher bezeichneten Reste die bei Formel I angegebene Bedeutung haben, worin jedoch

in Ia Ind Indol-3-yl, Methylindol-3-yl, Dimethylindol-3-yl, Methoxyindol-3-yl, Dimethoxy-indol-3-yl, Methylthioindol-3-yl, Hydroxyindol-3-yl, Dihydroxyindol-3-yl, Fluorindol-3-yl, Chlorindol-3-yl, Dichlorindol-3-yl, Bromindol-3-yl, Cyanindol-3-yl oder Methylendioxyindol-3-yl bedeutet, wobei die Substituenten vorzugsweise in 5- und/oder 6-Stellung stehen;

in Ib Ind Indol-3-yl, 5- oder 6-Methylindol-3-yl, 5,6-Dimethylindol-3-yl, 5- oder 6-Methoxy-indol-3-yl, 5,6-Dimethoxyindol-3-yl, 6-Methylthioindol-3-yl, 6-Hydroxyindol-3-yl, 5-Chlorindol-3-yl oder 5-Fluorindol-3-yl bedeutet;

in Ic Ind Indol-3-yl oder 5,6-Dimethoxyindol-3-yl bedeutet;

in Id A $-(CH_2)_4-$bedeutet;

in Ie A $-CH_2-L-CH_2CH_2-$ bedeutet;

in If A $-CH_2-S-CH_2-CH_2$ bedeutet;

in Ig Th 2-Thienyl bedeutet;

in Ih Ind Indol-3-yl, 5,6-Dimethylindol-3-yl, 6-Methoxyindol-3-yl, 5,6-Dimethoxy-indol-3-yl, 6-Methylthioindol-3-yl, 6-Hydroxyindol-3-yl, 5-Fluorindol-3-yl oder 5-Chlorindol-3-yl,
A $-(CH_2)_4-$ oder $-CH_2-S-CH_2CH_2-$ und
Th 2-Thienyl
bedeuten;

in Ii Ind Indol-3-yl oder 5,6-Dimethoxyindol-3-yl,
A $-(CH_2)_4-$ oder $-CH_2-S-CH_2CH_2-$ und
L 2-Thienyl
bedeuten;

in Ij Ind Indol-3-yl,
A $-(CH_2)_4-$ oder $-CH_2-S-CH_2CH_2-$ und
Th 2-Thienyl
bedeuten;

in Ik Ind Indol-3-yl oder 5,6-Dimethoxyindol-3-yl,
A $-(CH_2)_4-$ und
Th 2-Thienyl
bedeuten.

Die Verbindungen der Formel I können ein oder mehrere asymmetrische Kohlenstoffatome besitzen. Sie können daher als Racemate, falls mehrere asymmetrische Kohlenstoffatome vorhanden sind, auch als Gemische mehrere Racemate sowie in verschiedenen optisch-aktiven Formen vorliegen.

Gegenstand der Erfindung ist ferner ein Verfahren zur Herstellung der Verbindungen der Formel I sowie ihrer physiologisch unbedenklichen Säureadditionssalze, dadurch gekennzeichnet, dass man eine Verbindung der allgemeinen Formel

$$Ind-A-X^1 \qquad II,$$

worin
$X^1$ X oder $NH_2$ und
X Cl, Br, J, OH oder eine reaktionsfähige funktionell abgewandelte OH-Gruppe bedeuten und
Ind und A die angegebenen Bedeutungen haben, mit einer Verbindung der allgemeinen Formel III

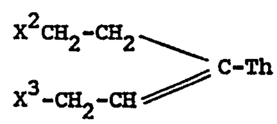

III,

worin
$X^2$ und $X^3$ gleich oder verschieden sein können und, falls $X^1 = NH_2$ ist, jeweils X, andernfalls zusammen NH bedeuten, und
Th die angegebene Bedeutung hat,
umsetzt oder dass man eine sonst der Formel I entsprechende Verbindung, die jedoch an Stelle eines oder mehrerer Wasserstoffatome eine oder mehrere reduzierbare Gruppe(n) und/oder eine oder mehrere zusätzliche C-C- und/oder C-N-Bindung(en) enthält, mit einem reduzierenden Mittel behandelt oder dass man eine sonst der Formel I entsprechende Verbindung, die jedoch an Stelle eines oder mehrerer Wasserstoffatome eine oder mehrere solvolytisch abspaltbare Gruppe(n) enthält, mit einem solvolysierenden Mittel behandelt oder dass man eine Verbindung der allgemeinen Formel IV

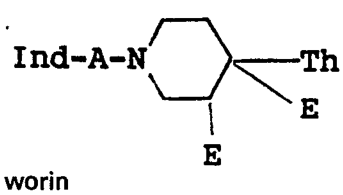

IV,

worin
ein Rest E X, CN oder $NH_2$,
der andere Rest E H bedeutet und
Ind, A, Th und X die angegebenen Bedeutungen haben, mit einem HE-abspaltenden Mittel behandelt
und/oder dass man gegebenenfalls in einer Verbindung der Formel I eine Thioethergruppe zu einer SO-Gruppe oder $SO_2$-Gruppe oder eine

$SO$-Gruppe zu einer $SO_2$-Gruppe oxydiert und/oder eine Alkoxygruppe unter Bildung einer OH-Gruppe spaltet und/oder dass man eine erhaltene Base der Formel I durch Behandeln mit einer Säure in eines ihrer physiologisch unbedenklichen Säureadditionssalze umwandelt.

Die Herstellung der Verbindungen der Formel I erfolgt im übrigen nach an sich bekannten Methoden, wie sie in der Literatur (z.B. in den Standardwerken wie Houben-Weyl, Methoden der Organischen Chemie, Georg-Thieme-Verlag, Stuttgart; Organic Reactions, John Wiley & Sons, Inc., New York) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, dass man sie aus dem Reaktionsgemisch nicht isoliert, sondern sofort weiter zu den Verbindungen der Formel I umsetzt.

In den Indolderivaten der Formel II ist $X^1$ vorzugsweise X; dementsprechend sind in den Verbindungen der Formel III $X^2$ und $X^3$ vorzugsweise zusammen NH. Der Rest X ist vorzugsweise Cl oder Br; er kann jedoch auch J, OH oder eine reaktionsfähig funktionell abgewandelte OH-Gruppe bedeuten, insbesondere Alkylsulfonyloxy mit 1–6 (z.B. Methansulfonyloxy) oder Arylsulfonyloxy mit 6–10 C-Atomen (z.B. Benzolsulfonyloxy, p-Toluolsulfonyloxy, 1- oder 2-Naphthalinsulfonyloxy).

Dementsprechend sind die Indolderivate der Formel I insbesondere durch Umsetzung der Verbindungen der Formeln Ind-A-Cl oder Ind-A-Br mit Tetrahydropyridinderivaten der Formel III, worin $X^2$ und $X^3$ zusammen eine NH-Gruppe bedeuten (nachstehend als IIIa bezeichnet) erhältlich.

Die Verbindungen der Formeln II und III sind zum Teil bekannt, die nicht bekannten Verbindungen der Formeln II und III können leicht analog zu den bekannten Verbindungen hergestellt werden.

Verbindungen der Formel II ($A = -CH_2-S-CH_2CH_2-$) können z.B. hergestellt werden aus Gramin und Thiolen der Formel $HS-CH_2CH_2X^1$, z.B. $HS-CH_2CH_2OH$. Die Sulfoxide und Sulfone der Formeln $Ind-CH_2-SO-CH_2CH_2X^1$ und $Ind-CH_2-SO_2-CH_2CH_2X^1$ sind durch Oxydation der Thioether der Formel $Ind-CH_2-S-CH_2CH_2X^1$ zugänglich. Primäre Alkohole der Formel II ($X^1 = OH$), z.B. $Ind-(CH_2)_4-OH$ oder $Ind-CH_2-S-CH_2CH_2OH$, sind z.B. durch Reduktion der entsprechenden Carbonsäuren oder ihrer Ester erhältlich. Behandeln mit Thionylchlorid, Bromwasserstoff, Phosphortribromid oder ähnlichen Halogenverbindungen liefert die entsprechenden Halogenide der Formel Ind-A-Hal. Die entsprechenden Sulfonyloxyverbindungen sind erhältlich aus den Alkoholen Ind-A-OH durch Umsetzung mit den entsprechenden Sulfonsäurechloriden.

Die Jodverbindungen der Formel Ind-A-J können z.B. durch Einwirkung von Kaliumjodid auf die zugehörigen p-Toluolsulfonsäureester hergestellt werden, die Amine der Formel $Ind-A-NH_2$ z.B. aus den Halogeniden mit Phthalimidkalium oder durch Reduktion der entsprechenden Nitrile.

Die Tetrahydropyridinderivate IIIa sind z.B. erhältlich durch Umsetzung von 4-Piperidon mit metallorganischen Verbindungen der Formel M-Th (worin M ein Li-Atom oder MgHal und Hal Cl, Br oder J bedeutet), anschliessende Hydrolyse zu den entsprechenden 4-Th-4-hydroxy-piperidinen sowie nachfolgende Dehydratisierung. Verbindungen der Formel III ($X^2$ und $X^3$ = jeweils X) sind z.B. herstellbar durch Reduktion von 3-Th-2-penten-1,5-disäure-diestern zu 3-Th-2-penten-1,5-diolen und gegebenenfalls anschliessende Umsetzung mit $SOCl_2$ bzw. $PBr_3$.

Die Umsetzung der Verbindungen II und III verläuft nach Methoden, wie sie für die Alkylierung von Aminen aus der Literatur bekannt sind. Man kann ohne Gegenwart eines Lösungsmittels die Komponenten miteinander verschmelzen, gegebenenfalls im geschlossenen Rohr oder im Autoklaven. Es ist aber auch möglich, die Verbindungen in Gegenwart eines indifferenten Lösungsmittels umzusetzen. Als Lösungsmittel eignen sich z.B. Kohlenwasserstoffe, wie Benzol, Toluol, Xylol; Ketone wie Aceton, Butanon; Alkohole wie Methanol, Ethanol, Isopropanol, n-Butanol; Ether wie Tetrahydrofuran (THF) oder Dioxan; Amide wie Dimethylformamid (DMF) oder N-Methylpyrrolidon; Nitrile wie Acetonitril, gegebenenfalls auch Gemische dieser Lösungsmittel untereinander oder Gemische mit Wasser. Der Zusatz eines säurebindenden Mittels, beispielsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylanilin, Pyridin oder Chinolin oder eines Überschusses der Aminkomponenten $Ind-A-NH_2$ bzw. des Piperidinderivates der Formel IIIa kann günstig sein.

Die Reaktionszeit liegt je nach den angewendeten Bedingungen zwischen einigen Minuten und 14 Tagen, die Reaktionstemperatur zwischen etwa 0 und 150°C, normalerweise zwischen 20 und 130°C.

Es ist ferner möglich, eine Verbindung der Formel I zu erhalten, indem man ein Vorprodukt, das an Stelle von Wasserstoffatomen eine oder mehrere reduzierbare Gruppe(n) und/oder eine oder mehrere zusätzliche C-C- und/oder C-N-Bindung(en) enthält, mit reduzierenden Mitteln behandelt, vorzugsweise bei Temperaturen zwischen −80 und +250°C in Gegenwart mindestens eines inerten Lösungsmittels.

Reduzierbare (durch Wasserstoff ersetzbare) Gruppen sind insbesondere Sauerstoff in einer Carbonylgruppe, Hydroxy, Arylsulfonyloxy (z.B. p-Toluolsulfonyloxy), N-Benzolsulfonyl, N-Benzyl oder O-Benzyl.

Es ist grundsätzlich möglich, Verbindungen,

die nur eine, oder solche, die nebeneinander zwei oder mehr der oben angeführten Gruppen bzw. zusätzlichen Bindungen enthalten, reduktiv in eine Verbindung der Formel I überzuführen. Vorzugsweise bedient man sich hierzu des nascierenden Wasserstoffs oder komplexer Metallhydride, ferner der Reduktion nach Wolff-Kishner.

Bevorzugte Ausgangsstoffe für die Reduktion entsprechen der Formel V

Ind'-A'-Q-Th V,

worin

Ind' einen Indol-3-ylrest, der ein- oder zweifach durch Alkyl, O-Alkyl, S-Alkyl, SO-Alkyl, $SO_2$-Alkyl, OH, F, Cl, Br, $CF_3$, CN und/oder O-Benzyl oder durch eine Methylendioxygruppe und/oder durch eine Arylsulfonylgruppe oder eine Benzylgruppe in 1-Stellung substituiert sein kann,

A' eine $-(CH_2)_4-$ oder $-CH_2-L-CH_2CH_2-$Kette, worin eine oder mehrere $-CH_2-$Gruppe(n) durch -CO-Gruppe(n) und/oder ein oder mehrere Wasserstoffatome durch OH-Gruppen ersetzt sein können,

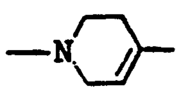 oder

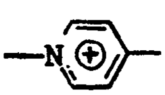 An$^{\ominus}$ und

An-$^{\ominus}$ ein Anion einer starken Säure bedeuten, worin jedoch nicht gleichzeitig Ind' = Ind, A' = A und

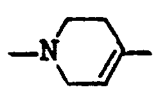 sein können.

In den Verbindungen der Formel V ist A' bevorzugt $-(CH_2)_3-CO-CH_2-$ oder $CH_2-L-CH_2-CO-$, ferner bevorzugt $-CH_2-CO-(CH_2)_2-$ oder $-CO-(CH_2)_3-$.

Verbindungen der Formel V sind z.B. herstellbar durch Umsetzung von 4-Th-1,2,3,6-tetrahydropyridinen oder 4-Th-pyridinen mit einer Verbindung der Formel VI

Ind'-A'-X[1] VI,

worin

Ind', A' und X[1] die angegebenen Bedeutungen haben, unter den Bedingungen, die oben für die Umsetzung von II mit III angegeben sind.

Die Herstellung von Säureamiden der Formel V (A' = $-(CH_2)_3-CO-$ oder $-CH_2-L-CH_2-CO-$,

Q = 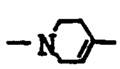 ),

die als Ausgangsstoffe für die Reduktion bevorzugt sind, gelingt z.B. aus den freien Carbonsäuren der Formeln Ind'$-(CH_2)_3-COOH$ bzw. Ind'$-CH_2-L-CH_2-COOH$ und Tetrahydropyridinen der Formel IIIa in Gegenwart eines Dehydratisierungsmittels wie Carbonyldiimidazol oder Dicyclohexylcarbodiimid in einem der angegebenen inerten Lösungsmittel, bevorzugt THF.

Wird bei der Herstellung der Verbindungen der Formel I durch Reduktion als Reduktionsmittel nascierender Wasserstoff verwendet, so kann man diesen z.B. durch Behandlung von Metallen mit schwachen Säuren oder mit Basen erzeugen. So kann man z.B. ein Gemisch von Zink mit Alkalilauge oder von Eisen mit Essigsäure verwenden.

Geeignet ist auch die Verwendung von Natrium oder einem anderen Alkalimetall in einem Alkohol wie Ethanol, Isopropanol, Butanol, Amyl- oder Isoamylalkohol, oder in Phenol. Man kann ferner eine Aluminium-Nickel-Legierung in alkalisch-wässriger Lösung, gegebenenfalls unter Zusatz von Ethanol, verwenden. Auch Natrium- oder Aluminiumamalgam in wässerig-alkoholischer oder wässeriger Lösung sind zur Erzeugung des nascierenden Wasserstoffs geeignet. Die Umsetzung kann auch in heterogener Phase durchgeführt werden, wobei man zweckmässig eine wässerige und eine Benzol- oder Toluol-Phase verwendet.

Als Reduktionsmittel können ferner besonders vorteilhaft komplexe Metallhydride, wie $LiAlH_4$, $NaBH_4$, Diisobutylaluminiumhydrid oder $NaAl(OCH_2CH_2OCH_3)_2H_2$ sowie Diboran eingesetzt werden, falls erwünscht unter Zusatz von Katalysatoren wie $BF_3$, $AlCl_3$ oder LiBr. Als Lösungsmittel eignen sich hierfür insbesondere Ether wie Diethylether, Di-n-butylether, THF, Dioxan, Diglyme oder 1,2-Dimethoxyethan sowie Kohlenwasserstoffe wie Benzol. Für eine Reduktion mit $NaBH_4$ sind in erster Linie Alkohole wie Methanol oder Ethanol, ferner Wasser sowie wässerige Alkohole als Lösungsmittel geeignet. Nach diesen Methoden reduziert man vorzugsweise bei Temperaturen zwischen -80 und +150°C, insbesondere zwischen etwa 0 und etwa 100°C.

Besonders vorteilhaft lassen sich -CO-Gruppen in Säureamiden mit $LiAlH_4$ in THF bei Temperaturen zwischen etwa 0 und 66°C zu $CH_2$-Gruppen reduzieren. Dabei können in 1-Stellung des Indolrings befindliche Arylsulfonyl-Schutzgruppen gleichzeitig reduktiv abgespalten werden.

Eine Reduktion der Pyridiniumsalze der Formel V (worin Q 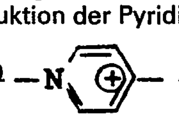 An-$^{\ominus}$ und An vorzugsweise Cl oder Br bedeutet) zu Verbindungen der Formel I gelingt z.B. mit $NaBH_4$ in Wasser, Methanol oder Ethanol oder in Gemischen dieser Lösungsmittel, falls erwünscht unter Zusatz einer Base wie NaOH, bei Temperaturen zwischen etwa 0 und 80°C.

N-Benzylgruppen können reduktiv mit Natrium in flüssigem Ammoniak abgespalten werden.

Es ist ferner möglich, eine oder mehrere Carbonylgruppen nach der Methode von Wolff-Kishner zu $CH_2$-Gruppen zu reduzieren, z.B. durch Behandlung mit wasserfreiem Hydrazin in absolutem Ethanol unter Druck bei Temperaturen zwischen etwa 150 und 250°C. Als Katalysator wird vorteilhaft Natriumalkoholat verwendet. Die Reduktion kann auch nach der Methode von Huang-Minlon variiert werden, indem man mit Hydrazinhydrat in einem hochsiedenden, mit Wasser mischbaren Lösungsmittel, wie Diethylenglykol oder Triethylenglykol, in Gegenwart von Alkali, wie Natriumhydroxid, umsetzt.

Das Reaktionsgemisch wird in der Regel etwa 3–4 Stunden gekocht. Anschliessend wird das Wasser abdestilliert und das gebildete Hydrazon bei Temperaturen bis zu etwa 200°C zersetzt. Die Wolff-Kishner-Reduktion kann auch bei Raumtemperatur in Dimethylsulfoxid mit Hydrazin ausgeführt werden.

Verbindungen, die sonst der Formel I entsprechen, aber an Stelle eines oder mehrerer H-Atome eine oder mehrere solvolytisch abspaltbare Gruppe(n) enthalten, können zu den Verbindungen der Formel I solvolysiert, insbesondere hydrolysiert werden. Die Ausgangsstoffe für die Solvolyse sind beispielsweise erhältlich durch Reaktion von IIIa mit Verbindungen, die der Formel II ($X^1 = X$) entsprechen, aber an Stelle eines oder mehrerer H-Atome eine oder mehrere solvolytisch abspaltbare Gruppe(n) enthalten. So können 1-Acylindolderivate (entsprechend der Formel I, aber in 1-Stellung des Ind-Restes eine Acylgruppe enthaltend, vorzugsweise eine Alkanoyl-, Alkylsulfonyl- oder Arylsulfonylgruppe mit jeweils bis zu 10 C-Atomen, wie Methan-, Benzol- oder p-Toluolsulfonyl) zu den entsprechenden in der 1-Stellung des Indolringes unsubstituierten Indolderivaten hydrolysiert werden, z.B. in saurem, besser in neutralem oder alkalischem Medium bei Temperaturen zwischen 0 und 200°C.

Als basische Katalysatoren verwendet man zweckmässig Natrium-, Kalium- oder Calciumhydroxid, Natrium- oder Kaliumcarbonat, oder Ammoniak. Als Lösungsmittel wählt man vorzugsweise Wasser; niedere Alkohole wie Methanol, Ethanol; Ether wie THF, Dioxan; Sulfone wie Tetramethylensulfon; oder deren Gemische, besonders die Wasser enthaltenden Gemische. Eine Hydrolyse kann auch bereits beim Behandeln mit Wasser allein erfolgen, insbesondere in der Siedehitze.

Man gelangt ferner zu Verbindungen der Formel I, indem man aus Verbindungen der Formel IV unter Ausbildung einer Doppelbindung HE abspaltet. Entsprechend der Definition von E kann es sich z.B. handeln um eine Abspaltung von Halogenwasserstoff, Wasser (Dehydratisierung), einer Carbonsäure oder einer anderen Säure, von Ammoniak oder von HCN. Die Ausgangsstoffe der Formel IV sind z.B. erhältlich durch Umsetzung von II ($X^1 = X$) mit einer Verbindung der Formel VII

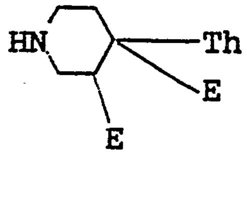

VII

worin E und Th die angegebenen Bedeutungen haben. Falls einer der Reste E = Hal ist, kann dieser Substituent unter basischen Reaktionsbedingungen leicht eliminiert werden. Als Basen können verwendet werden: Alkalimetallhydroxide, Alkalimetallcarbonate, Alkoholate, wie z.B. Kalium-tert.-butylat, Amine, wie z.B. Dimethylanilin, Pyridin, Collidin oder Chinolin; als Lösungsmittel benutzt man z.B. Benzol, Toluol, Cyclohexan, Methanol, Dioxan, THF oder tert.-Butanol. Die als Basen verwendeten Amine können auch im Überschuss als Lösungsmittel eingesetzt werden. Bedeutet der eine der Reste E eine OH-Gruppe, so benutzt man als wasserabspaltende Mittel vorzugsweise Säuren wie Essigsäure, Salzsäure oder Gemische beider. Der Zusatz eines Lösungsmittels (z.B. Wasser oder Ethanol) kann von Vorteil sein. Die Eliminierung von Acyl-, Alkylsulfonyl- sowie Alkoxysulfonyl-oxy- oder Amino-Resten kann unter ähnlichen Bedingungen durchgeführt werden. Eine Eliminierung von Sulfonsäure-Resten, z.B. die der Mesylate oder Tosylate, erfolgt schonend durch Kochen in DMF oder Dimethylsulfoxid mit Alkalimetallcarbonaten, z.B. $Li_2CO_3$, oder mit Kaliumacetat.

Ammoniak kann bereits durch Erhitzen der Salze der entsprechenden Aminoverbindungen (insbesondere der 4-Aminoderivate) abgespalten werden. In ähnlicher Weise kann HCN aus Verbindungen der Formel IV (eine Gruppe E = CN) durch Erhitzen abgespalten werden. Die Eliminierung von HE aus IV erfolgt allgemein bei Temperaturen zwischen etwa 0 und etwa 250°, vorzugsweise zwischen 50 und 200°C.

Weiterhin kann man in einem Thioether der Formel I die Thioethergruppe zu einer SO-Gruppe oder zu einer $SO_2$-Gruppe oder in einem Sulfoxid der Formel I die SO-Gruppe zu einer $SO_2$-Gruppe oxydieren. Die zu oxydierenden Thioether- oder Sulfoxidgruppen können in der Gruppe A und/oder als Substituenten im Rest Ind vorhanden sein. Will man die Sulfoxide erhalten, so oxydiert man beispielsweise mit Wasserstoffperoxid, Persäuren wie m-Chlorperbenzoesäure, Cr(VI)-Verbindungen wie Chromsäure, $KMnO_4$, 1-Chlorbenztriazol, Ce(IV)-Verbindungen wie $(NH_4)_2Ce(NO_3)_6$, negativ substituierten aromatischen Diazoniumsalzen wie o- oder p-Nitrophenyldiazoniumchlorid oder elektrolytisch unter verhältnismässig milden Bedingungen und bei relativ niedrigen Temperaturen (etwa −80 bis +100°C). Will man dagegen die Sulfone (aus den Thioethern oder den Sulfoxiden) erhalten, so verwendet man die gleichen Oxydationsmittel unter kräftigeren Bedingungen und/oder im Überschuss sowie in der Regel bei höheren Temperaturen. Bei diesen Umsetzungen können die üblichen inerten Lösungsmittel zugegen oder abwesend sein. Als inerte Lösungsmittel eignen sich beispielsweise Wasser, wässerige Mineralsäuren, wässerige Alkalilaugen, niedere Alkohole wie Methanol oder Ethanol, Ester wie Ethylacetat, Ketone wie Aceton, niedere Carbonsäuren wie Essigsäure, Nitrile wie Acetonitril, Kohlenwasserstoffe wie Benzol, chlorierte Kohlenwasserstoffe wie Chloroform oder $CCl_4$.

Ein bevorzugtes Oxydationsmittel ist 30%iges wässeriges Wasserstoffperoxid. Dieses führt bei Anwendung der berechneten Menge in Lösungsmitteln wie Essigsäure, Aceton, Methanol, Ethanol oder wässeriger Natronlauge bei Temperaturen zwischen -20 und 100°C zu den Sulfoxiden, im Überschuss bei höheren Temperaturen, vorzugsweise in Essigsäure oder in einem Gemisch aus Essigsäure und Acetanhydrid, zu den Sulfonen.

Ether der Formel I, in denen der Rest Ind ein- oder zweifach durch O-Alkyl substituiert ist, können nach Methoden, die aus der Literatur bekannt sind, gespalten werden, wobei die entsprechenden Hydroxyderivate entstehen. Z.B. kann man die Ether spalten durch Behandeln mit HBr oder HJ in wässeriger oder essigsaurer Lösung, durch Erhitzen mit Lewis-Säuren wie $AlCl_3$ oder Bortrihalogeniden oder durch Verschmelzen mit Pyridin- oder Anilin-hydrohalogeniden, vorzugsweise Pyridinhydrochlorid, bei etwa 150-250°C.

Eine erhaltene Base der Formel I kann mit einer Säure in das zugehörige Säureadditionssalz übergeführt werden. Für diese Umsetzung eignen sich Säuren, die physiologisch unbedenkliche Salze liefern. So können anorganische Säuren verwendet werden, z.B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäure, Laurylschwefelsäure.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze. Diese Zubereitungen können als Arzneimittel in der Human- oder Veterinärmedizin eingesetzt werden. Als Trägersubstanzen kommen organische oder anorganische Stoffe in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlehydrate wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositorien, zur parenteralen Applikation Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden.

Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Gegenstand der Erfindung ist ferner die Verwendung der Verbindungen der Formel I und ihrer physiologisch unbedenklichen Salze bei der therapeutischen Behandlung des menschlichen oder tierischen Körpers und bei der Bekämpfung von Krankheiten, insbesondere von Parkinsonismus, von extrapyramidalen Störungen bei der Neuroleptikatherapie, von Depressionen und/oder Psychosen und von Nebenwirkungen bei der Behandlung der Hypertonie (z.B. mit $\alpha$-Methyldopa). Ferner können die Verbindungen in der Endokrinologie und Gynäkologie Verwendung finden, z.B. zur Therapie von Akromegalie, Hypogonadismus, sekundärer Amenorrhoe, prämenstruellem Syndrom, unerwünschter puerperaler Laktation und generell als Prolaktin-Hemmer, weiterhin zur Therapie cerebraler Störungen (z.B. Migräne), insbesondere in der Geriatrie ähnlich wie gewisse Ergot-Alkaloide.

Dabei werden die erfindungsgemässen Substanzen in der Regel in Analogie zu bekannten, im Handel befindlichen Präparaten (z.B. Bromocriptin, Dihydroergocornin) verabreicht, vorzugsweise in Dosierungen zwischen etwa 0,2 und 500 mg, insbesondere zwischen 0,2 und 50 mg pro Dosierungseinheit. Die tägliche Dosierung liegt vorzugsweise zwischen etwa 0,001 und 10 mg/kg Körpergewicht. Die niedrigen Dosierungen (etwa 0,2 bis 1 mg pro Dosierungseinheit; etwa 0,001 bis 0,005 mg/kg Körpergewicht) kommen dabei insbesondere für die Verwendung als Migränemittel in Betracht; für die übrigen Indikationen werden Dosierungen zwischen 10 und 50 mg pro Dosierungseinheit bevorzugt. Die spezielle Dosis für jeden bestimmten Patienten hängt jedoch von

den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

In den nachfolgenden Beispielen bedeutet «übliche Aufarbeitung»:

Man gibt, falls erforderlich, Wasser hinzu, extrahiert mit einem organischen Lösungsmittel wie Toluol, Chloroform oder Dichlormethan, trennt ab, trocknet die organische Phase über Natriumsulfat, filtriert, dampft ein und reinigt durch Chromatographie und/oder Kristallisation. Temperaturen sind in Celsiusgraden angegeben. Rf-Werte wurden an Kieselgel erhalten (Laufmittel: Dichlormethan/Cyclohexan/Ethylacetat/Methanol 3,2 : 3,2 : 1,6 : 2, wenn nicht anders angegeben).

Beispiel 1

Man rührt eine Lösung von 2,08 g 3-(4-Chlorbutyl)-indol [oder 2,52 g 3-(4-Brombutyl)-indol] und 1,65 g 4-(2-Thienyl)-1,2,3,6-tetrahydropyridin [Hydrochlorid, F. 242°C; erhältlich durch Reaktion von 2-Thienyl-Li mit 4-Oxo-1-piperidincarbonsäureethylester zu 4-Hydroxy-4-(2-thienyl)-1-piperidincarbonsäureethylester, alkalische Abspaltung der Schutzgruppe und Dehydratisierung mit Salzsäure] in 10 ml Acetonitril 12 Stunden bei 20°C, arbeitet wie üblich auf und erhält 3-[4-(4-(2-Thienyl)-1,2,3,6-tetrahydropyridyl)-butyl]-indol; Hydrochlorid («T»), Rf 0,52.

Analog erhält man aus den entsprechenden Chlor- oder Bromalkyl- bzw. Chlor- oder Bromthiaalkyl-indolen:

3-[4-(4-(2-Thienyl)-1,2,3,6-tetrahydropyridyl)-butyl]-2-methylindol
3-[4-(4-(2-Thienyl)-1,2,3,6-tetrahydropyridyl)-butyl]-5-methoxyindol, F. 105–107°C
3-[4-(4-(2-Thienyl)-1,2,3,6-tetrahydropyridyl)-butyl]-6-methoxyindol, F. 158°C
3-[4-(4-(2-Thienyl)-1,2,3,6-tetrahydropyridyl)-butyl]-5-methylthioindol
3-[4-(4-(2-Thienyl)-1,2,3,6-tetrahydropyridyl)-butyl]-6-methylthioindol, Hydrochlorid, F. 208°C
3-[4-(4-(2-Thienyl)-1,2,3,6-tetrahydropyridyl)-butyl]-6-methylsulfinylindol
3-[4-(4-(2-Thienyl)-1,2,3,6-tetrahydropyridyl)-butyl]-6-methylsulfonylindol
3-[4-(4-(2-Thienyl)-1,2,3,6-tetrahydropyridyl)-butyl]-4-hydroxyindol
3-[4-(4-(2-Thienyl)-1,2,3,6-tetrahydropyridyl)-butyl]-5-hydroxyindol, F. 200–202°C
3-[4-(4-(2-Thienyl)-1,2,3,6-tetrahydropyridyl)-butyl]-6-hydroxyindol, F. 181°C
3-[4-(4-(2-Thienyl)-1,2,3,6-tetrahydropyridyl)-butyl]-5-fluorindol, Hydrochlorid, Rf 0,58
3-[4-(4-(2-Thienyl)-1,2,3,6-tetrahydropyridyl)-butyl]-5-chlorindol, Hydrochlorid, Rf 0,59
3-[4-(4-(2-Thienyl)-1,2,3,6-tetrahydropyridyl)-butyl]-7-bromindol
3-[4-(4-(2-Thienyl)-1,2,3,6-tetrahydropyridyl)-butyl]-5-trifluormethylindol
3-[4-(4-(2-Thienyl)-1,2,3,6-tetrahydropyridyl)-butyl]-5-cyanindol
3-[4-(4-(2-Thienyl)-1,2,3,6-tetrahydropyridyl)-butyl]-5,6-dimethylindol, Hydrochlorid, Rf 0,50 (Chloroform:Methanol = 9:1)
3-[4-(4-(2-Thienyl)-1,2,3,6-tetrahydropyridyl)-butyl]-5,6-dimethoxyindol, F. 113–115°C
3-[4-(4-(2-Thienyl)-1,2,3,6-tetrahydropyridyl)-butyl]-5,6-dichlorindol
3-[4-(4-(2-Thienyl)-1,2,3,6-tetrahydropyridyl)-butyl]-5,6-methylendioxyindol
3-[4-(4-(2-Thienyl) - 1,2,3,6-tetrahydropyridyl)-2-thia-butyl]-indol, F. 86–87°C; Hydrochlorid, F. 182–184°C
3-[4-(4-(2-Thienyl)-1,2,3,6-tetrahydropyridyl)-2-thia-butyl]-2-methylindol
3-[4-(4-(2-Thienyl)-1,2,3,6-tetrahydropyridyl)-2-thia-butyl]-5-methoxyindol
3-[4-(4-(2-Thienyl)-1,2,3,6-tetrahydropyridyl)-2-thia-butyl]-6-methoxyindol
3-[4-(4-(2-Thienyl)-1,2,3,6-tetrahydropyridyl)-2-thia-butyl]-5-methylthioindol
3-[4-(4-(2-Thienyl)-1,2,3,6-tetrahydropyridyl)-2-thia-butyl]-6-methylthioindol
3-[4-(4-(2-Thienyl)-1,2,3,6-tetrahydropyridyl)-2-thia-butyl]-6-methylsulfinylindol
3-[4-(4-(2-Thienyl)-1,2,3,6-tetrahydropyridyl)-2-thia-butyl]-6-methylsulfonylindol
3-[4-(4-(2-Thienyl)-1,2,3,6-tetrahydropyridyl)-2-thia-butyl]-4-hydroxyindol
3-[4-(4-(2-Thienyl)-1,2,3,6-tetrahydropyridyl)-2-thia-butyl]-5-hydroxyindol
3-[4-(4-(2-Thienyl)-1,2,3,6-tetrahydropyridyl)-2-thia-butyl]-6-hydroxyindol
3-[4-(4-(2-Thienyl)-1,2,3,6-tetrahydropyridyl)-2-thia-butyl]-5-fluorindol
3-[4-(4-(2-Thienyl)-1,2,3,6-tetrahydropyridyl)-2-thia-butyl]-5-chlorindol
3-[4-(4-(2-Thienyl)-1,2,3,6-tetrahydropyridyl)-2-thia-butyl]-7-bromindol
3-[4-(4-(2-Thienyl)-1,2,3,6-tetrahydropyridyl)-2-thia-butyl]-5-trifluormethylindol
3-[4-(4-(2-Thienyl)-1,2,3,6-tetrahydropyridyl)-2-thia-butyl]-5-cyanindol
3-[4-(4-(2-Thienyl)-1,2,3,6-tetrahydropyridyl)-2-thia-butyl]-5,6-dimethylindol
3-[4-(4-(2-Thienyl)-1,2,3,6-tetrahydropyridyl)-2-thia-butyl]-5,6-dimethoxyindol
3-[4-(4-(2-Thienyl)-1,2,3,6-tetrahydropyridyl)-2-thia-butyl]-5,6-dichlorindol
3-[4-(4-(2-Thienyl)-1,2,3,6-tetrahydropyridyl)-2-thia-butyl]-5,6-methylendioxyindol.

Beispiel 2

Ein Gemisch von 4,43 g 3-(4-p-Toluolsulfonyloxybutyl)-indol und 3,3 g 4-(2-Thienyl)-1,2,3,6-tetra-hydropyridin wird auf 130°C erhitzt. Nach Abklingen der exothermen Reaktion und Erkalten arbeitet man wie üblich auf und erhält «T».

Analog erhält man aus den entsprechenden Tosylaten:

3-[4-(4-(2-Thienyl)-1,2,3,6-tetrahydropyridyl)-butyl]-5-methylindol

3-[4-(4-(2-Thienyl)-1,2,3,6-tetrahydropyridyl)-butyl]-6-methylindol
3-[4-(4-(2-Thienyl)-1,2,3,6-tetrahydropyridyl)-butyl]-5-butylindol
3-[4-(4-(2-Thienyl)-1,2,3,6-tetrahydropyridyl)-butyl]-5-ethoxyindol
3-[4-(4-(2-Thienyl)-1,2,3,6-tetrahydropyridyl)-butyl]-5-butoxyindol
3-[4-(4-(2-Thienyl)-1,2,3,6-tetrahydropyridyl)-butyl]-6-butylthioindol
3-[4-(4-(2-Thienyl)-1,2,3,6-tetrahydropyridyl)-butyl]-5-bromindol.

Beispiel 3

Man kocht 2,99 g 3-(4-Jodbutyl)-indol, 1,65 g 4-(2-Thienyl)-1,2,3,6-tetrahydropyridin und 1,5 g wasserfreies Kaliumcarbonat in 25 ml n-Butanol 2 Stunden unter Rühren, lässt erkalten, arbeitet wie üblich auf und erhält «T».

Beispiel 4

Ein Gemisch von 1,88 g 3-(4-Aminobutyl)-indol und 2,21 g 1,5-Dichlor-3-(2-thienyl)-2-penten (erhältlich durch Reduktion von 3-(2-thienyl)-2-penten-1,5-disäurediethylester mit $LiAlH_4$ und anschliessende Reaktion mit $SOCl_2$) in 40 ml Aceton und 40 ml Wasser wird 24 Stunden gekocht und wie üblich aufgearbeitet. Man erhält «T».

Analog erhält man aus den entsprechenden Aminen und den entsprechenden 1,5-Dichlor-3-(2-thienyl)-2-pentenen die anderen in den Beispielen 1 und 2 angegebenen Verbindungen der Formel I.

Beispiel 5

Zu einer Lösung von 4,13 g 1-[4-(3-Indolyl)-butyl]-4-(2-thienyl)-pyridinium-bromid [erhältlich aus 3-(4-Brombutyl)-indol und 4-(2-Thienyl)-pyridin] in 50 ml 1n NaOH gibt man unter Rühren 1 g $NaBH_4$ in 20 ml Wasser und rührt danach noch 3 Stunden bei 60°C. Nach üblicher Aufarbeitung erhält man «T».

Analog erhält man durch Reduktion der entsprechenden Pyridiniumbromide die anderen in den Beispielen 1 und 2 angegebenen Verbindungen der Formel I.

Beispiel 6

Zu einer Suspension von 0,38 g $LiAlH_4$ in 10 ml THF tropft man eine Lösung von 3,50 g 3-[4-Oxo-4-(4-(2-thienyl)-1,2,3,6-tetrahydropyridyl)-butyl]-indol [erhältlich aus 4-(3-Indolyl)-Buttersäure und 4-(2-Thienyl)-1,2,3,6-tetrahydropyridin in Gegenwart von Carbonyldiimidazol in THF] in 10 ml THF unter Rühren. Nach Abklingen der Reaktion gibt man 5 ml Ethylacetat hinzu, arbeitet wie üblich auf und erhält «T».

Analog erhält man aus den entsprechenden Säureamiden, z.B.

3-[4-Oxo-4-(4-(2-thienyl)- 1,2,3,6-tetrahydropyridyl)-butyl]-2-methylindol
3-[4-Oxo-4-(4-(2-thyenyl)-1,2,3,6-tetrahydropyridyl)-butyl]-5-methoxyindol
3-[4-Oxo-4-(4-(2-thienyl)-1,2,3,6-tetrahydropyridyl)-butyl]-6-methoxyindol [Rf 0,5 (Chloroform: Methanol = 9:1)]
3-[4-Oxo-4-(4-(2-thienyl)-1,2,3,6-tetrahydropyridyl)-butyl]-5-methylthioindol
3-[4-Oxo-4-(4-(2-thienyl)-1,2,3,6-tetrahydropyridyl)-butyl]-6-methylthioindol (F. 218°C)
3-[4-Oxo-4-(4-(2-thienyl)-1,2,3,6-tetrahydropyridyl)-butyl]-6-methylsulfinylindol
3-[4-Oxo-4-(4-(2-thienyl)-1,2,3,6-tetrahydropyridyl)-butyl]-6-methylsulfonylindol
3-[4-Oxo-4-(4-(2-thienyl)-1,2,3,6-tetrahydropyridyl)-butyl]-4-hydroxyindol
3-[4-Oxo-4-(4-(2-thienyl)-1,2,3,6-tetrahydropyridyl)-butyl]-5-hydroxyindol
3-[4-Oxo-4-(4-(2-thienyl)-1,2,3,6-tetrahydropyridyl)-butyl]-6-hydroxyindol [Rf 0,48 (Chloroform: Methanol = 9:1)]
3-[4-Oxo-4-(4-(2-thienyl)-1,2,3,6-tetrahydropyridyl)-butyl]-5-fluorindol
3-[4-Oxo-4-(4-(2-thienyl)-1,2,3,6-tetrahydropyridyl)-butyl]-7-bromindol
3-[4-Oxo-4-(4-(2-thienyl)-1,2,3,6-tetrahydropyridyl)-butyl]-5-trifluormethylindol
3-[4-Oxo-4-(2-thienyl)-1,2,3,6-tetrahydropyridyl)-butyl]-5,6-dimethylindol (F. 193°C)
3-[4-Oxo-4-(4-(2-thienyl)-1,2,3,6-tetrahydropyridyl)-butyl]-5,6-dimethoxyindol
3-[4-Oxo-4-(4-(2-thienyl)-1,2,3,6-tetrahydropyridyl)-butyl]-5,6-dichlorindol
3-[4-Oxo-4-(4-(2-thienyl)-1,2,3,6-tetrahydropyridyl)-butyl]-5,6-methylendioxyindol
3-[4-Oxo-4-(4-(2-thienyl)-1,2,3,6-tetrahydropyridyl)-2-thiabutyl]-indol (F. 114–115°C)
sowie die anderen in den Beispielen 1 und 2 angegebenen Verbindungen der Formel I.

Beispiel 7

Man kocht 4,76 g 1-Benzolsulfonyl-3-[4-(4-(2-Thienyl) -1,2,3,6-tetrahydropyridyl) -butyl]-indol [erhältlich aus 1-Benzolsulfonyl-3-(4-chlor-butyl)-indol und 4-(2-Thienyl)-1,2,3,6-tetrahydropyridin] mit 1 g KOH in 7 ml Wasser und 14 ml Ethanol 16 Stunden, konzentriert das Gemisch, arbeitet wie üblich auf und erhält «T».

Beispiel 8

Man erhitzt 3,80 g 1-Methyl-3-[4-hydroxy-4-(2-thienyl)-1-piperidyl)-2-thiabutyl]-indol [erhältlich durch Reaktion von 1-Methyl-3-(4-brom-2-thiabutyl)-indol mit 4-Piperidon, anschliessende Umsetzung mit 2-Thienyl-Li und Hydrolyse] mit 40 ml in Salzsäure 2 Stunden auf 50°C, arbeitet wie üblich auf und erhält 1-Methyl-3-[4-(4-(2-thienyl)-1,2,3,6-tetrahydropyridyl)-2-thiabutyl]-indol.

Beispiel 9

Zu einer siedenden Lösung von 3,54 g 3-[4-(4-(2-Thienyl) -1,2,3,6-tetrahydropyridyl) -2-thiabutyl]-indol in 50 ml Ethanol gibt man 6 ml 30%iges $H_2O_2$ und kocht anschliessend 3 Stunden. Nach Zugabe weiterer 4 ml des Oxydationsmittels kocht man noch 9 Stunden, kühlt ab, arbeitet wie üblich auf und erhält 4-[4-(4-(2-Thienyl)-1,2,3,6-tetrahydropyridyl)-2-thiabutyl]-indol-S-oxid.

Analog sind durch Oxydation der entsprechenden Thioether erhältlich:

3-[4-(4-(2-Thienyl)-1,2,3,6-tetrahydropyridyl)-2-thiabutyl]-2-methylindol-S-oxid
3-[4-(4-(2-Thienyl)-1,2,3,6-tetrahydropyridyl)-2-thiabutyl]-5-methoxyindol-S-oxid
3-[4-(4-(2-Thienyl)-1,2,3,6-tetrahydropyridyl)-2-thiabutyl]-6-methoxyindol-S-oxid
3-[4-(4-(2-Thienyl)-1,2,3,6-tetrahydropyridyl)-2-thiabutyl]-5-methylsulfinylindol-S-oxid
3-[4-(4-(2-Thienyl)-1,2,3,6-tetrahydropyridyl)-2-thiabutyl]-6-methylsulfinylindol-S-oxid
3-[4-(4-(2-Thienyl)-1,2,3,6-tetrahydropyridyl)-2-thiabutyl]-4-hydroxyindol-S-oxid
3-[4-(4-(2-Thienyl)-1,2,3,6-tetrahydropyridyl)-2-thiabutyl]-5-hydroxyindol-S-oxid
3-[4-(4-(2-Thienyl)-1,2,3,6-tetrahydropyridyl)-2-thiabutyl]-6-hydroxyindol-S-oxid
3-[4-(4-(2-Thienyl)-1,2,3,6-tetrahydropyridyl)-2-thiabutyl]-5-fluorindol-S-oxid
3-[4-(4-(2-Thienyl)-1,2,3,6-tetrahydropyridyl)-2-thiabutyl]-5-chlorindol-S-oxid
3-[4-(4-(2-Thienyl)-1,2,3,6-tetrahydropyridyl)-2-thiabutyl]-7-bromindol-S-oxid
3-[4-(4-(2-Thienyl)-1,2,3,6-tetrahydropyridyl)-2-thiabutyl]-5-trifluormethylindol-S-oxid
3-[4-(4-(2-Thienyl)-1,2,3,6-tetrahydropyridyl)-2-thiabutyl]-5-cyanindol-S-oxid
3-[4-(4-(2-Thienyl)-1,2,3,6-tetrahydropyridyl)-2-thiabutyl]-5,6-dimethylindol-S-oxid
3-[4-(4-(2-Thienyl)-1,2,3,6-tetrahydropyridyl)-2-thiabutyl]-5,6-dimethoxyindol-S-oxid
3-[4-(4-(2-Thienyl)-1,2,3,6-tetrahydropyridyl)-2-thiabutyl]-5,6-dichlorindol-S-oxid
3-[4-(4-(2-Thienyl)-1,2,3,6-tetrahydropyridyl)-2-thiabutyl]-5,6-methylendioxyindol-S-oxid.

Beispiel 10

Zu einer Lösung von 3,54 g 3-[4-(4-(2-Thienyl)-1,2,3,6-tetrahydropyridyl)-2-thiabutyl]-indol in 20 ml Essigsäure gibt man 9 ml 30%iges $H_2O_2$ und kocht 90 Minuten. Nach der üblichen Aufarbeitung erhält man 3-[4-(4-(2-Thienyl)-1,2,3,6-tetrahydropyridyl)-2-thiabutyl]-indol-S-oxid.

Analog sind durch Oxydation der entsprechenden Thioether erhältlich:

3-[4-(4-(2-Thienyl)-1,2,3,6-tetrahydropyridyl)-2-thiabutyl]-2-methylindol-S,S-dioxid
3-[4-(4-(2-Thienyl)-1,2,3,6-tetrahydropyridyl)-2-thiabutyl]-5-methoxyindol-S,S-dioxid
3-[4-(4-(2-Thienyl)-1,2,3,6-tetrahydropyridyl)-2-thiabutyl]-6-methoxyindol-S,S-dioxid
3-[4-(4-(2-Thienyl)-1,2,3,6-tetrahydropyridyl)-2-thiabutyl]-5-methylsulfonylindol-S,S-dioxid
3-[4-(4-(2-Thienyl)-1,2,3,6-tetrahydropyridyl)-2-thiabutyl]-6-methylsulfonylindol-S,S-dioxid
3-[4-(4-(2-Thienyl)-1,2,3,6-tetrahydropyridyl)-2-thiabutyl]-4-hydroxyindol-S,S-dioxid
3-[4-(4-(2-Thienyl)-1,2,3,6-tetrahydropyridyl)-2-thiabutyl]-5-hydroxyindol-S,S-dioxid
3-[4-(4-(2-Thienyl)-1,2,3,6-tetrahydropyridyl)-2-thiabutyl]-6-hydroxyindol-S,S-dioxid
3-[4-(4-(2-Thienyl)-1,2,3,6-tetrahydropyridyl)-2-thiabutyl]-5-fluorindol-S,S-dioxid
3-[4-(4-(2-Thienyl)-1,2,3,6-tetrahydropyridyl)-2-thiabutyl]-5-chlorindol-S,S-dioxid
3-[4-(4-(2-Thienyl)-1,2,3,6-tetrahydropyridyl)-2-thiabutyl]-7-bromindol-S,S-dioxid
3-[4-(4-(2-Thienyl)-1,2,3,6-tetrahydropyridyl)-2-thiabutyl]-5-trifluormethylindol-S,S-dioxid
3-[4-(4-(2-Thienyl)-1,2,3,6-tetrahydropyridyl)-2-thiabutyl]-5-cyanindol-S,S-dioxid
3-[4-(4-(2-Thienyl)-1,2,3,6-tetrahydropyridyl)-2-thiabutyl]-5,6-dimethylindol-S,S-dioxid
3-[4-(4-(2-Thienyl)-1,2,3,6-tetrahydropyridyl)-2-thiabutyl]-5,6-dimethoxyindol-S,S-dioxid
3-[4-(4-(2-Thienyl)-1,2,3,6-tetrahydropyridyl)-2-thiabutyl]-5,6-dichlorindol-S,S-dioxid
3-[4-(4-(2-Thienyl)-1,2,3,6-tetrahydropyridyl)-2-thiabutyl]-5,6-methylendioxyindol-S,S-dioxid.

Beispiel 11

Ein Gemisch aus 4,03 g 3-[4-(4-(2-Thienyl)-1,2,3,6-tetrahydropyridyl) -butyl] -6-methoxyindol-hydrochlorid und 3,5 g Pyridinhydrochlorid wird 3 Stunden bei 160°C gerührt. Nach üblicher Aufarbeitung erhält man 3-[4-(4-(2-Thienyl)-1,2,3,6-tetrahydropyridyl)-butyl]-6-hydroxyindol, F. 181°C.

Die nachstehenden Beispiele betreffen pharmazeutische Zubereitungen, die Amine der Formel I oder ihre Säureadditionssalze enthalten:

Beispiel A: Tabletten

Ein Gemisch von 1 kg «T», 4 kg Lactose, 1,2 kg Kartoffelstärke, 0,2 kg Talk und 0,1 kg Magnesiumstearat wird in üblicher Weise zu Tabletten verpresst, derart, dass jede Tablette 10 mg Wirkstoff enthält.

Beispiel B: Dragees

Analog Beispiel A werden Tabletten gepresst, die anschliessend in üblicher Weise mit einem Überzug aus Saccharose, Kartoffelstärke, Talk, Tragant und Farbstoff überzogen werden.

Beispiel C: Kapseln

2 kg «T» werden in üblicher Weise in Hartgelatinekapseln gefüllt, so dass jede Kapsel 20 mg des Wirkstoffes enthält.

Beispiel D: Ampullen

Eine Lösung von 1 kg «T» in 30 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

Analog sind Tabletten, Dragees, Kapseln und Ampullen erhältlich, die einen oder mehrere der übrigen Wirkstoffe der Formel I und/oder ihre physiologisch unbedenklichen Säureadditionssalze enthalten.

**Patentansprüche**

1. Indolderivate der allgemeinen Formel I

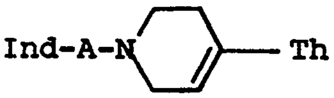

I

worin

Ind einen Indol-3-ylrest, der ein- oder zweifach durch Alkyl, -O-Alkyl, -S-Alkyl, -SO-Alkyl, $-SO_2$-Alkyl, OH, F, Cl, Br, $CF_3$ und/oder CN oder durch eine Methylendioxygruppe substituiert sein kann,

A $-(CH_2)_4-$ oder $-CH_2-L-CH_2CH_2-$,

L -S-, -SO- oder $-SO_2-$ und

Th einen 2- oder 3-Thienylrest

bedeuten und

worin die Alkylgruppen jeweils 1–4 C-Atome besitzen,

sowie deren physiologisch unbedenkliche Säureadditionssalze.

2. Als Verbindungen gemäss Anspruch 1:

a) 3-[4-(4-(2-Thienyl)-1,2,3,6-tetrahydropyridyl)-butyl]-indol.

b) 3-[4-(4-(2-Thienyl)-1,2,3,6-tetrahydropyridyl)-butyl]-5,6-dimethoxyindol.

c) 3-[4-(4-(2-Thienyl)-1,2,3,6-tetrahydropyridyl)-2-thiabutyl]-indol.

3. Verfahren zur Herstellung von Indolderivaten der allgemeinen Formel I

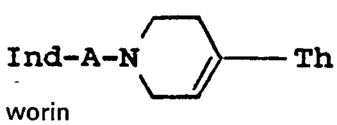

I ,

worin

Ind einen Indol-3-ylrest, der ein- oder zweifach durch Alkyl, -O-Alkyl, -S-Alkyl, -SO-Alkyl, $-SO_2$-Alkyl, OH, F, Cl, Br, $CF_3$ und/oder CN oder durch eine Methylendioxygruppe substituiert sein kann,

A $-(CH_2)_4-$ oder $-CH_2-L-CH_2CH_2-$,

L -S-, -SO- oder $-SO_2-$ und

Th einen 2- oder 3-Thienylrest

bedeuten und

worin die Alkylgruppen jeweils 1–4 C-Atome besitzen,

sowie von deren physiologisch unbedenklichen Säureadditionssalzen,

dadurch gekennzeichnet, dass man entweder eine Verbindung der allgemeinen Formel II

$$Ind-A-X^1 \qquad II,$$

worin

$X^1$ X oder $NH_2$ und

X Cl, Br, J, OH oder eine reaktionsfähig funktionell abgewandelte OH-Gruppe bedeuten und

Ind und A die angegebenen Bedeutungen haben,

mit einer Verbindung der allgemeinen Formel III

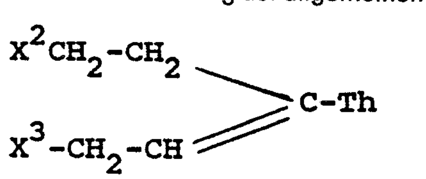

III,

worin

$X^2$ und $X^3$ gleich oder verschieden sein können und, falls $X^1 = NH_2$ ist, jeweils X, andernfalls zusammen NH bedeuten und

Th die angegebene Bedeutung hat,

umsetzt,

oder dass man eine sonst der Formel I entsprechende Verbindung, die jedoch an Stelle eines oder mehrerer Wasserstoffatome eine oder mehrere reduzierbare Gruppe(n) und/oder eine oder mehrere zusätzliche C-C- und/oder C-N-Bindung(en) enthält, mit einem reduzierenden Mittel behandelt oder dass man eine sonst der Formel I entsprechende Verbindung, die jedoch an Stelle eines oder mehrerer Wasserstoffatome eine oder mehrere solvolytisch abspaltbare Gruppe(n) enthält, mit einem solvolysierenden Mittel behandelt oder dass man eine Verbindung der Formel IV

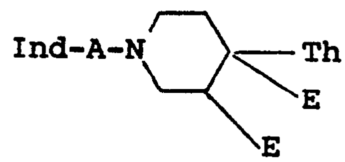

IV,

worin

ein Rest E X, CN oder $NH_2$,

der andere Rest E H bedeutet und

Ind, A, Th und X die angegebenen Bedeutungen haben,

mit einem HE-abspaltenden Mittel behandelt und/oder dass man gegebenenfalls in eine Verbindung der Formel I eine Thioethergruppe zu einer SO-Gruppe oder $SO_2$-Gruppe oder eine SO-Gruppe zu einer $SO_2$-Gruppe oxydiert und/oder eine Alkoxygruppe unter Bildung einer OH-Gruppe spaltet und/oder dass man eine erhaltene Base der Formel I durch Behandeln mit einer Säure in eines ihrer physiologisch unbedenklichen Säureadditionssalze umwandelt.

4. Verfahren zur Herstellung pharmazeutischer Zubereitungen, dadurch gekennzeichnet, dass man eine Verbindung der Formel I und/oder eines ihrer physiologisch unbedenklichen Säureadditionssalze zusammen mit mindestens einem festen, flüssigen oder halbflüssigen Träger- oder Hilfsstoff und ggf. in Kombination mit einem oder mehreren weiteren Wirkstoffen in eine geeignete Dosierungsform bringt.

5. Pharmazeutische Zubereitung, gekennzeichnet durch einen Gehalt an mindestens einer Verbindung der allgemeinen Formel I und/oder einem ihrer physiologisch unbedenklichen Säureadditionssalze.

6. Verbindungen der allgemeinen Formel I nach Patentanspruch 1 zur Bekämpfung von Krankheiten.

7. Verwendung von Verbindungen der allgemeinen Formel I nach Patentanspruch 1 und/oder ihrer physiologisch unbedenklichen Säureadditionssalze zur Herstellung von Arzneimitteln.

**Claims**

1. Indole derivatives of the general formula I

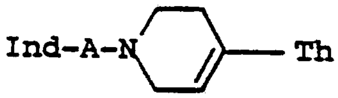

I

in which Ind is a 3-indolyl radical which can be substituted once or twice by alkyl, O-alkyl, S-alkyl, SO-alkyl, $SO_2$-alkyl, OH, F, Cl, Br, $CF_3$ and/or CN or by a methylenedioxy group, A is $-(CH_2)_4-$ or $-CH_2-L-CH_2CH_2-$, L is -S-, -SO- or $-SO_2-$, and Th is a 2- or 3-thienyl radical, and in which the alkyl groups each have 1-4 C atoms, and their physiologically acceptable acid addition salts.

2. As compounds according to Claim 1:

a) 3-[4-(4-(2-Thienyl)-1,2,3,6-tetrahydropyridyl)-butyl]indole.
b) 3-[4-(4-(2-Thienyl)-1,2,3,6-tetrahydropyridyl)-butyl]-5,6-dimethoxyindole.
c) 3-[4-(4-(2-Thienyl)-1,2,3,6-tetrahydropyridyl)-2-thiabutyl]indole.

3. Process for the preparation of indole derivatives of the general formula I

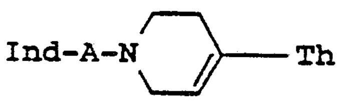

I

in which Ind is a 3-indolyl radical which can be substituted once or twice by alkyl, O-alkyl, S-alkyl, SO-alkyl, $SO_2$-alkyl, OH, F, Cl, Br, $CF_3$ and/or CN or by a methylenedioxy group, A is $-(CH_2)_4-$ or $-CH_2-L-CH_2CH_2-$, L is -S-, -SO- or $-SO_2-$, and Th is a 2- or 3-thienyl radical, and in which the alkyl groups each have 1-4 C atoms, and of their physiologically acceptable acid addition salts, characterised in that either a compound of the general formula II

$Ind-A-X^1$ II

in which $X^1$ is X or $NH_2$, and X is Cl, Br, I, OH or a reactive derivative of an OH group, and Ind and A have the indicated meanings, is reacted with a compound of the general formula III

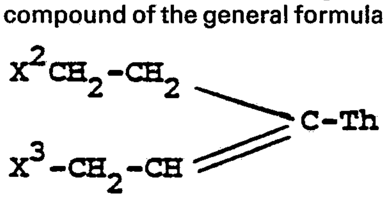

III

in which $X^2$ and $X^3$ can be identical or different and, when $X^1$ is $NH_2$, are each X, otherwise are together NH, and Th has the indicated meaning, or in that a compound which otherwise corresponds to the formula I but, in place of one or more hydrogen atoms, contains one or more reducible group(s) and/or one or more additional C-C and/or C-N bond(s) is treated with a reducing agent, or in that a compound which otherwise corresponds to the formula I but, in place of one or more hydrogen atoms, contains one or more group(s) which can be eliminated by solvolysis is treated with a solvolysing agent, or in that a compound of the formula IV

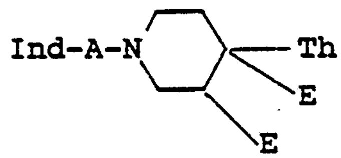

IV

in which one radical E is X, CN or $NH_2$, the other radical E is H, and Ind, A, Th and X have the indicated meanings, is treated with an agent eliminating He, and/or in that, where appropriate, in a compound of the formula I, a thioether group is oxidised to an SO group or $SO_2$ group, or an SO group is oxidised to an $SO_2$ group, and/or an alkoxy group is cleaved to form an OH group, and/or in that a base of the formula I which has been obtained is converted, by treatment with an acid, into one of its physiologically acceptable acid addition salts.

4. Process for the preparation of pharmaceutical formulations, characterised in that a compound of the formula I and/or one of its physiologically acceptable acid addition salts is converted into a suitable dosage form together with at least one solid, liquid or semiliquid vehicle or auxiliary and, where appropriate, combined with one or more other active compounds.

5. Pharmaceutical formulation, characterised by containing at least one compound of the general formula I and/or one of its physiologically acceptable acid addition salts.

6. Compounds of the general formula I according to Patent Claim 1 for controlling diseases.

7. Use of compounds of the general formula I according to Patent Claim 1 and/or their physiologically acceptable acid addition salts for the preparation of medicaments.

**Revendications**

1. Dérivés de l'indole répondant à la formule générale I

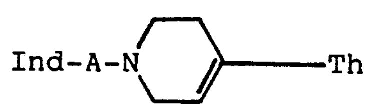

I

dans laquelle

Ind représente un groupe indole-3-yle qui peut porter un ou deux substituants alkyle, -O-alkyle, -S-alkyle, -SO-alkyle, $-SO_2$-alkyle, OH, F, Cl, Br, $CF_3$ et/ou CN ou un substituant méthylène-dioxy,

A représente $-(CH_2)_4-$ ou $-CH_2-L-CH_2CH_2-$,

L représente -S-, -SO-, ou $-SO_2-$, et

Th représente un groupe 2- ou 3-thiényle,

dans lesquels les groupes alkyle contiennent chacun de 1 à 4 atomes de carbone, et leurs sels acceptables pour l'usage pharmaceutique formés par addition avec des acides.

2. En tant que composés selon la rev. 1:
a) le 3-[4-(4-(2-thiényl)-1,2,3,6-tétrahydropyridyl)-butyl]-indole,
b) le 3-[4-(4-(2-thiényl)-1,2,3,6-tétrahydropyridyl)-butyl]-5,6-diméthoxyindole,
c) le 3-[4-(4-(2-thiényl)-1,2,3,6-tétrahydropyridyl)-2-thiabutyl]-indole.

3. Procédé de préparation des dérivés de l'indole répondant à la formule générale I

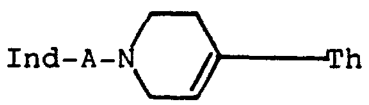 I

dans laquelle
Ind représente un groupe indole-3-yle qui peut porter un ou deux substituants alkyle, -O-alkyle, -S-alkyle, -SO-alkyle, $-SO_2$-alkyle, OH, F, Cl, Br, $CF_3$ et/ou CN ou porter un substituant méthylène-dioxy,
A représente $-(CH_2)_4-$ ou $-CH_2-L-CH_2CH_2-$,
L représente -S-, -SO-, ou $-SO_2-$, et
Th représente un groupe 2- ou 3-thiényle,
dans lesquels les groupes alkyle contiennent chacun 1 à 4 atomes de carbone, et de leurs sels acceptables pour l'usage pharmaceutique formés par addition avec des acides, caractérisé en ce que:
on fait réagir un composé de formule générale II

$Ind-A-X^1$ II

dans laquelle
$X^1$ représente X ou $NH_2$, et
X représente Cl, Br, I, OH ou un groupe OH ayant subi une modification fonctionnelle et réactif et
Ind et A ont les significations indiquées ci-dessus, avec un composé de formule générale III

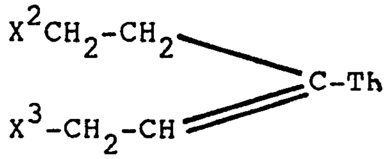 III

dans laquelle
$X^2$ et $X^3$, ayant des significations identiques ou différentes, ont chacun les mêmes significations que X lorsque $X^1 = NH_2$, et dans les autres cas forment ensemble un groupe NH, et
Th a les significations indiquées ci-dessus, ou bien,
partant d'un composé qui répondrait par ailleurs à la formule I mais porte un ou plusieurs groupes réductibles et/ou une ou plusieurs liaisons supplémentaires C-C et/ou C-N à la place d'un ou plusieurs atomes d'hydrogène, on traite ce composé par un agent réducteur, ou bien partant d'un composé qui répondrait par ailleurs à la formule I mais qui contient un ou plusieurs groupes éliminables par solvolyse à la place d'un ou plusieurs atomes d'hydrogène, on traite ce composé par un agent solvolysant, ou bien
on traite un composé de formule IV

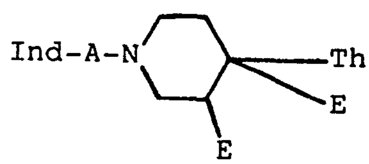 IV

dans laquelle
l'un des symboles E représente X, CN ou $NH_2$,
l'autre symbole E représente H, et
Ind, A, Th et X ont les significations indiquées ci-dessus, par un agent éliminant HE, et/ou le cas échéant, dans un composé de formule I, on oxyde un groupe thioéther en un groupe SO ou en un groupe $SO_2$ ou un groupe SO en un groupe $SO_2$ et/ou on élimine un groupe alcoxy avec formation d'un groupe OH et/ou on convertit une base obtenue répondant à la formule I en sel acceptable pour l'usage pharmaceutique par traitement à l'aide d'un acide.

4. Procédé de préparation des compositions pharmaceutiques, caractérisé en ce que l'on met sous une forme de dosage appropriée un composé de formule I et/ou l'un de ses sels acceptables pour l'usage pharmaceutique formé par addition avec un acide, avec au moins un véhicule ou produit auxiliaire solide, liquide ou semiliquide et le cas échéant, en combinaison avec une ou plusieurs autres substances actives.

5. Composition pharmaceutique caractérisée en ce qu'elle contient au moins un composé de formule générale I et/ou l'un de ses sels acceptables pour l'usage pharmaceutique formés par addition avec des acides.

6. Composés de formule générale I selon la rev. 1, pour la lutte contre les maladies.

7. Utilisation des composés de formule générale I selon la rev. 1 et/ou de leurs sels acceptables pour l'usage pharmaceutique formés par addition avec des acides pour la préparation de médicaments.